(19)

Europäisches
Patentamt

European
Patent Office

Office européen
des brevets

(11) **EP 3 388 139 A1**

(12) **EUROPEAN PATENT APPLICATION**

(43) Date of publication:
**17.10.2018 Bulletin 2018/42**

(21) Application number: **17166680.3**

(22) Date of filing: **13.04.2017**

(51) Int Cl.:
**B01D 61/28** (2006.01)          **B01D 63/02** (2006.01)
**B01D 69/08** (2006.01)          **A61M 1/16** (2006.01)
**B01D 61/24** (2006.01)          B01D 71/42 (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Gambro Lundia AB
220 10 Lund (SE)**

(72) Inventors:
• DONATO, Danilo
**87036 Rende-Cosenza (IT)**
• ELOOT, Sunny
**9800 Deinze (BE)**

• ZWEIGART, Carina
**72355 Schoemberg (DE)**
• KOLB, Michael
**72181 Starzach (DE)**
• BOSCHETTI-DE-FIERRO, Adriana
**72379 Hechingen (DE)**
• KRAUSE, Bernd
**72414 Rangendingen (DE)**
• STORR, Markus
**70794 Filderstadt (DE)**

(74) Representative: **Hornung, Veronika Margot
c/o Gambro Dialysatoren GmbH
Holger-Crafoord-Strasse 26
72379 Hechingen (DE)**

(54) **OPTIMIZED HEMODIALYZER FOR BLOOD PURIFICATION**

(57)     The present disclosure concerns a hemodialyzer for the purification of blood and use thereof. The hemodialyzer comprises a bundle of hollow fiber membranes, wherein the hollow fiber membranes have an inner diameter ($d_B$) within the range of 168-175 $\mu$m, a fiber wall thickness ($\delta_m$) within the range of 25-26 $\mu$m, and an effective length (L) within the hemodialyzer within the range of 207-287 mm. The bundle of hollow fiber membranes has a packing density within the range of 57.5-60.0%, and a total membrane surface area ($A_{tot}$) within the range of from 1.64 to 1.73 m$^2$.

Figure 1

**EP 3 388 139 A1**

**Description**

**Technical Field**

The present invention relates to a dialyzer comprising a bundle of semipermeable hollow fiber membranes which is suitable for blood purification, wherein the dialyzer has a design which provides an increased ability to remove solutes during blood purification. The invention also relates to using said dialyzer in hemodialysis.

**Description of Related Art**

Capillary dialyzers are widely used for blood purification in patients suffering from renal insufficiency, i.e. for treatment of the patients by hemodialysis, hemodiafiltration or hemofiltration. The devices generally consist of a casing comprising a tubular section with end caps capping the mouths of the tubular section. A bundle of hollow fiber membranes is arranged in the casing in way that a seal is provided between the first flow space formed by the fiber cavity and a second flow space surrounding the membranes on the outside. Examples of such devices are disclosed in EP 0 844 015 A2, EP 0 305 687 A1 and WO 01/60477 A2.

Module performance is controlled by membrane properties and mass transfer boundary layers that develop in the fluid adjacent to the membrane surface in the hollow fiber lumen and the outer shell surface. Boundary layer resistances are significant in many processes including dialysis. Accordingly, one important factor influencing performance in the device is the hollow fiber membrane which is used for accomplishing the device. Dialysis membranes today are designed to allow for the removal of uremic toxins and excess water from the blood of patients with chronic renal failure while balancing the electrolyte content in the blood with the dialysis fluid.

Another important factor influencing performance of the device depends strongly on the geometry of the housing and the fiber bundle located therein, including the geometry of the single hollow fibers. Relevant parameters as concerns the fibers are, apart from their specific membrane structure, composition and related performance, the effective (accessible) length (L) of the fibers, the inner diameter ($d_B$) of the fibers, the wall thickness ($\delta_m$) of the fibers and their overall three-dimensional geometry. The aforementioned concentration and thermal boundary layers adjacent to the fiber surface as well as uniformity of the flow through a dialyzer will otherwise be influenced by the packing density. "Crimping" or "undulating" the fibers also contributes to the flow characteristics of a dialyzer. Crimping or undulating the single hollow fibers transforms a straight fiber into a generally wavy fiber. Crimped fibers overcome problems of uniformity of flow around and between the fibers and of longitudinal fiber contact which can reduce the fiber surface area available for mass transfer by reducing said longitudinal contact between adjacent fibers, thereby improving flow uniformity and access to membrane area. As mentioned before, the performance of dialyzers is also related to the membrane packing density which in turn is closely connected to the flow characteristics. A high membrane packing density increases the performance of the device as long as the uniformity of the flow is not affected.

The sieving property of a membrane as such, irrespective of the overall performance of the dialyzer, i.e. the membrane's permeability to solutes, is determined by the pore size and sets the maximum size for the solutes that can be dragged through the membrane with the fluid flow. The sieving coefficient for a given substance could be simply described as the ratio between the substance concentration in the filtrate and its concentration in the feed (i.e. the blood or plasma), and is therefore a value between 0 and 1. Assuming that the size of a solute is proportional to its molecular weight, a common way to illustrate the properties of membranes is by building a sieving curve, which depicts the sieving coefficient as a function of the molecular weight. The expression "molecular weight cut-off" or "MWCO" used herein is a value for describing the retention capabilities of a membrane and refers to the molecular mass of a solute where the membranes have a rejection of 90%, corresponding to a sieving coefficient of 0.1. The MWCO can alternatively be described as the molecular mass of a solute, such as, for example, dextrans or proteins were the membrane allow passage of 10% of the molecules. The shape of the curve depends, to a significant extent, on the pore size distribution and to the physical form of appearance of the membrane and its pore structure, which can otherwise be only inadequately described. Sieving coefficients therefore are a good description not only of the performance of a membrane but are also descriptive of the specific submacroscopic structure of the membrane. To enhance membrane characterization, the molecular weight retention onset (MWRO) is another now established parameter which is used for better characterizing membranes. By using both MWCO and MWRO it becomes evident how membranes really perform. The MWRO is defined as the molecular weight at which the sieving coefficient is 0.9. It is otherwise analogous to the MWCO but describes when the sieving coefficient starts to fall. Defining two points on the sieving curves allows a better, more concise characterization of the sigmoid curve, giving an indication of the pore sizes and also of the pore size distribution and thus of the most relevant physical parameters which determine a membrane. The expression "molecular weight retention onset", "MWRO" or "nominal molecular weight retention onset" therefore refers to the molecular mass of a solute where the membranes have a rejection of 10%, or, in other words, allow passage of 90% of the solute, corresponding to a sieving coefficient of 0.9. The dextran data from molecular weight fractions is also directly related to the size of the molecules and is an indirect measure of the pore sizes in the membranes. Thus, the MWRO is also directly related to a physical property of the membrane. One can interpret this value as some reference of where the pore size distribution starts, while the MWCO indicates where it ends. The use of dextran sieving curves together with the respective MWCO and MWRO values based thereon allows differentiating the existing dialyzer types low-flux, high-flux, medium cut-off, protein leaking or high cut-off.

Conventional dialysis membranes can be classified as low-flux or high-flux, depending on their permeability. A third group, called protein leaking membranes, is also available on some markets. These three membrane groups were described in a review by Ward in 2005 (Ward RA. Protein-leaking membranes for hemodialysis: a new class of membranes in search of an application? J Am Soc Nephrol. 2005;16(8):2421-2430). High-flux membranes used in devices, such as, for example, Polyflux® 170H (Gambro), Revaclear® MAX (Gambro), Ultraflux® EMIC2 (Fresenius Medical Care), Optiflux® F180NR (Fresenius Medical Care), FXCorDiax™ (Fresenius Medical Care), have been on the market for several years now. The high-flux membranes used therein are mainly polysulfone or polyethersulfone based membranes and methods for their production have been described, for example, in US 5,891,338 and EP 2 113 298. Another known membrane is used in the Phylther® HF 17G filter from Bellco Società unipersonale a r.l. It is generally referred to as high-flux membrane and is based on polyphenylene. In polysulfone or polyethersulfone based membranes such as mentioned above, the polymer solution typically comprises between 10 and 20 weight-% of polyethersulfone or polysulfone as hydrophobic polymer and 2 to 11 weight-% of a hydrophilic polymer, in most cases PVP, wherein said PVP consists of a low and a high molecular PVP component. The resulting high-flux type membranes generally consist of 80-99% by weight of said hydrophobic polymer and 1-20% by weight of said hydrophilic polymer. During production of the membrane the temperature of the spinneret often is in the range of from 20-55°C. Polymer combinations, process parameters and performance data can otherwise be retrieved from the references mentioned or can be taken from publicly available data sheets. The expression "high-flux membrane(s)" as used herein refers to membranes having a MWRO between 5 kDa and 10 kDa and a MWCO between 25 kDa and 65 kDa, as determined by dextran sieving measurements according to Boschetti et al. (2013). The average pore radius is in the range of from 3.5 to 5.5 nm, wherein the pore size is determined from the MWCO based on dextran sieving coefficients according to Boschetti-de-Fierro et al. (2013) and Granath et al.: Molecular weight distribution analysis by gel chromatography on sephadex. J Chromatogr A. 1967;28(C):69-81. The main difference between high-flux membranes and low-flux membranes is a higher water permeability and the ability to remove small-to-middle molecules like β2-microglobulin.

Protein leaking membranes, on the other hand, have a water permeability similar to that of low-flux membranes, the ability to remove small-to-middle molecules similar to high-flux membranes, but show a loss of higher molecular weight proteins, such as albumin and larger molecules.

A fourth type of membranes, called high cut-off membranes, has first been disclosed in WO 2004/056460 A1 wherein certain early high cut-off membranes are described which were designed for for the treatment of sepsis in dialyzers by eliminating sepsis-associated inflammatory mediators. Advanced dialyzers making use of high cut-off membranes which are currently on the market are, for example, HCO1100®, septeX™ and Theralite®, all available from Gambro Lundia AB. Known uses of high cut-off membranes include treatment of sepsis, chronic inflammation (EP 2 161 072 A1), amyloidosis and rhabdomyolysis and treatment of anemia (US 2012/0305487 A1), the most explored therapy to date being the treatment of myeloma kidney (US 7,875,183 B2). The expression "high cut-off membrane" or "high cut-off membranes" as used herein refers to membranes having a MWRO of between 15 and 20 kDa and a MWCO of between 170-320 kDa. The membranes can also be characterized by a pore radius, on the selective layer surface of the membrane, of between 8-12 nm. For the avoidance of doubt, the determination of MWRO and MWCO for a given membrane is according to the methods of Boschetti-de-Fierro et al. (2013). Accordingly, the expressions "as determined by dextran sieving" or "based on dextran sieving" also refer to the dextran sieving method as described in Boschetti-de-Fierro et al. (2013). Processes for producing high cut-off membranes have been described, for example, in the aforementioned references. As disclosed already in WO 2004/056460 A1, a key element for their generation is an increase in the temperature of the spinning process, i.e. the temperature of the spinneret, the spinning shaft temperature and temperature of the coagulation bath, relative to the spinning conditions for producing a high-flux membrane with about the same composition of polymers. In addition, for the production of the latest high cut-off membranes such as the Theralite® membrane, the ratio of water and solvent (H₂O/solvent) in the polymer solution is also slightly changed to lower values while the polymer content in said solution can otherwise be similar to or the same as used for producing high-flux membranes such as, for example, the Revaclear® MAX membrane.

Now entering the market is another, new class of membranes with enhanced sieving properties, allowing removal of middle and large uremic solutes which cannot be addressed by high cut-off membranes described above during acceptable albumin losses for chronic patients. These membranes are referred to as Medium Cut-Off membranes (MCO membranes), and are characterized by a molecular retention onset (MWRO) of between 8.5 kDa and 14.0 kDa and a molecular weight cut-off (MWCO) of between 55 kDa and 130 kDa as determined by dextran sieving curves before the membrane has had contact with blood or a blood product. As a result, these membranes significantly extend the removable range of uremic solutes while sufficiently retaining albumin for safe use in chronic applications with patients suffering from renal failure. Such membranes and dialyzers comprising same, as well as methods for preparing such membranes are disclosed in e.g. WO2015/118045 and WO 2015/118046. Also these membranes, like most of the high-flux membranes, are polysulphone-based, polyethersulphone-based or poly(aryl)ether-sulfone-based synthetic membranes, comprising, in addition, a hydrophilic component such as, for example, PVP and optionally low amounts of further polymers, such as, for example, polyamide or polyurethane.

While investigations on the characteristics of membrane types and their performance are very advanced, a systematic analysis of the effect of all the dialyzer-related factors especially including the physical parameters mentioned above on solute clearance has not been reported in literature. This makes it difficult to optimize dialyzer design and operation to maximize solute removal from patient's blood in a directed, less empiric manner. As discussed before, it is known that solute transport in dialyzers is strongly affected by dialyzer geometry, membrane properties and operating conditions. Optimization of membrane modules with in vitro experiments is limited by the difficult prediction of the extent to which membrane transport properties and module fluid dynamics influence solute clearance. Mathematical models of solute transport in dialyzers can help to determine how module design and operating conditions affect dialyzer efficiency and developing optimized membranes and modules. A systematic and in-depth analysis of the influence of the dialyzer-related factors accounting for module geometry, membrane transport properties and operating conditions on solute transport has not been reported yet. The inventors have set out, therefore, to identify the most relevant dialyzer-related factors determining dialyzer efficiency and to investigate how their interplay influences solute clearance in order to optimize dialyzer design. The inventors are thereby providing a novel set of interconnected physical parameters for an optimal dialyzer design which can be used for devising feasible dialyzers with optimized clearance characteristics.

## Summary

It was the object of the present invention to develop an optimized hemodialyzer for the purification of blood, allowing maximized solute clearance.

According to the invention this and other objects are achieved, in full or at least in part, by a hemodialyzer for the purification of blood comprising a bundle of synthetic hollow fiber membranes having an inner diameter ($d_B$) within the range of 168-175 $\mu$m, and a fiber wall thickness ($\delta_m$) within the range of 25-26 $\mu$m, wherein the fiber membranes have an effective length (L) within the hemodialyzer within the range of 207-287 mm, and wherein the bundle of hollow fiber membranes has a packing density within the range of 57.5-60.0%, and wherein the total membrane surface area ($A_{tot}$) is in the range of from 1.64 to 1.73 m$^2$.

With these specific geometric properties, the present inventors have found that increased clearance, as exemplified by urea clearance and myoglobin clearance, may be accomplished in comparison with prior art dialyzers.

In other embodiments of the invention, the optimized hemodialyzers may further be defined by certain dimensionless parameters, specifically the membrane pressure modulus ($\alpha$), mainly accounting for membrane transport properties, which may be in a range of from 0.31 to 0.40. Another dimensionless parameter which may define the hemodialyzer is the maximal reduced Peclet number for urea ($Pe_{r,max,\ urea}$), which may be in the range of from 0.45 to 0.49 at a blood flow ($Q_B$) of 500 ml/min.

In one embodiment the hemodialyzer has a housing with an inner diameter ($d_H$) within the range of 31-35 mm.

In another embodiment the hemodialyzer membranes have a molecular retention onset (MWRO) of between 5 kDa and 20 kDa and a molecular weight cut-off (MWCO) of between 25 kDa and 320 kDa as determined by dextran sieving before blood contact to the membrane.

In another embodiment the hemodialyzer membranes have a molecular retention onset (MWRO) of between 5 kDa and 10 kDa and a molecular weight cut-off (MWCO) of between 25 kDa and 65 kDa as determined by dextran sieving before blood contact to the membrane.

In another embodiment the hemodialyzer membranes have a molecular retention onset (MWRO) of between 9 kDa and 14 kDa and a molecular weight cut-off (MWCO) of between 55 kDa and 130 kDa as determined by dextran sieving before blood contact to the membrane.

In another embodiment the hemodialyzer membranes have a molecular retention onset (MWRO) of between 15 kDa and 20 kDa and a molecular weight cut-off (MWCO) of between 170 kDa and 320 kDa as determined by dextran sieving before blood contact to the membrane.

In a further embodiment, the housing of the hemodialyzer has an inner total volume ($V_{tot}$) within the range of 195 to 199 ml.

In even a further embodiment, the fiber bundle within the hemodialyzer consists of 80-95 % of crimped fibers and of 5% to 15% non-crimped fibers, relative to the total number of fibers in the bundle.

In one embodiment the hollow fiber membranes within the hemodialyzer have an inner diameter ($d_B$) within the range of 170-174 $\mu$m.

In another embodiment the hollow fiber membranes within the hemodialyzer have a fiber wall thickness ($\delta_m$) of 25 $\mu$m.

In a further embodiment, the bundle of hollow fiber membranes has a packing density of from 58.0% to 60.0% within the hemodialyser.

In one embodiment of the present invention, the hollow fiber membrane comprises at least one hydrophobic polymer and at least one hydrophilic polymer. The at least one hydrophobic polymer may be chosen from the group consisting of poly(aryl)ethersulphone (PAES), polypropylene (PP), polysulphone (PSU), polycarbonate (PC), polyacrylonitrile (PAN), polyamide (PA), polytetrafluorethylene (PTFE), polyethersulphone (PES), or combination thereof, and the at least one hydrophilic polymer may be chosen from the group consisting of polyvinylpyrrolidone (PVP), polyethyleneglycol (PEG), polyvinylalcohol (PVA), and a copolymer of polypropyleneoxide and polyethyleneoxide (PPO-PEO) or combinations thereof.

In one embodiment of the present invention, the hollow fiber membrane may comprise a copolymer of acrylonitrile and sodium methallyl sulfonate.

In one embodiment of the present invention, the hollow fiber membrane may comprise at least one hydrophobic polymer and at least one hydrophilic polymer, wherein the hydrophobic polymer may be chosen from the group consisting of polyarylethersulfone (PAES) or polysulfone (PS), and the at least one hydrophilic polymer may be polyvinylpyrrolidone (PVP).

The present invention is also directed to methods of using the hemodialyzer in blood purification applications, in particular in hemodialysis methods used to treat advanced and permanent kidney failure.

Other objectives, features and advantages of the present invention will appear from the following detailed disclosure, from the attached claims, as well as from the drawings. It is noted that the invention relates to all possible combinations of features.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

As used herein, the term "comprising" and variations of that term are not intended to exclude other additives, components, integers or steps.

In the context of the present invention, the expressions "hemodialyzer(s)", "hemodialysis device", "hemodialysis filter", "filter for hemodialysis" or "filter device for hemodialysis" are used synonymously and refer to the devices according to the invention as described herein. The expression "hemodiafilter(s)" as used herein refers to filter devices which can be used or are preferably used in blood treatments performed in hemodiafiltration methods for blood purification. The expressions "dialyzer", "dialysis filter", "filter" or "filter device", if not indicated otherwise, generally refer to devices which can be used for blood purification.

The expression "hemodialysis" as used herein refers to a primarily diffusive-type blood purification method wherein the differences in concentration drive the removal of uremic toxins and their passage through the dialyzer membrane which separates the blood from the dialysate. The expression "hemodiafiltration" as used herein refers to a blood purification method that combines diffusion and convection, wherein convection is achieved by applying a positive pressure gradient across the dialyzer membrane.

The expression "$V_{tot}$" as used herein refers to the inner total volume of the housing of the hemodialyzer.

The expression "Peclet number for urea" as used herein refers to Pe for the certain solute *urea* at a blood flow ($Q_B$) of 500 ml/min.

The hemodialyzers now accomplished are further characterized by clearance rates, determined according to ISO8637:2014(E), that in hemodialysis mode achieve values which can be achieved with prior art dialyzers only in hemodiafiltration mode, i.e. by applying a positive pressure gradient across the dialyzer membrane.

**Brief Description of the Drawings**

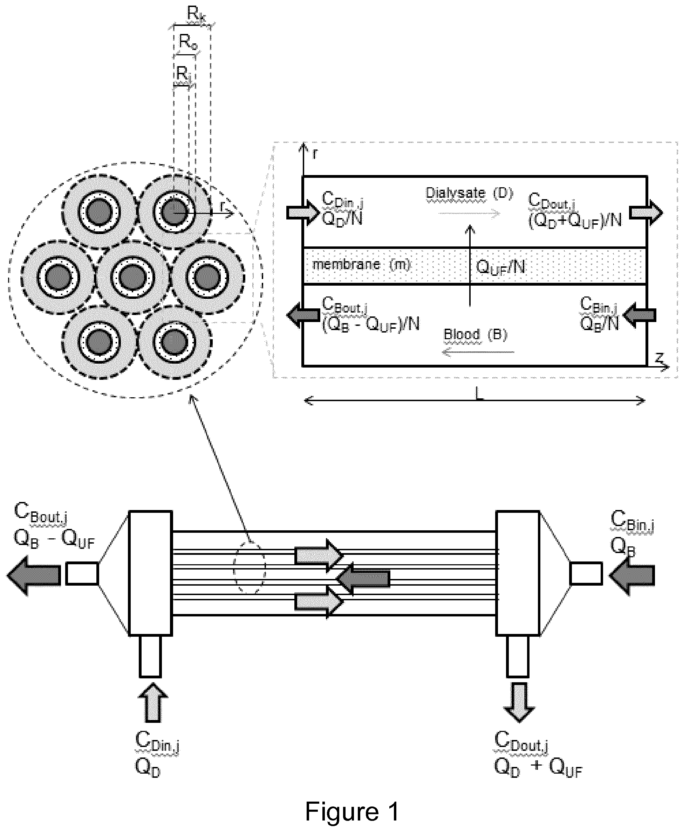

Figure 1 is a schematic drawing of a hemodialyzer. A bundle of around 10,000 hollow fibers is contained in an external housing. Blood flows inside the lumen of the hollow fibers while dialysate, an aqueous electrolyte solution, flows countercurrently around the fibers. Solute transport across the fiber semi-permeable membrane and between the blood and dialysate, occurs both by diffusion and convection. For models used to define the dialyzer of the invention, it is assumed that the fibers are evenly spaced and arranged in regular hexagonal arrays, and each fiber is surrounded by a uniform annulus. The interstitial spaces among neighbouring annuli are neglected. The solutes exemplarily considered are urea (molecular weight MW 60Da), creatinine (MW 113Da), phosphate (MW 95Da), β2-microglobulin (MW 11000Da) and myoglobin (MW16700Da).

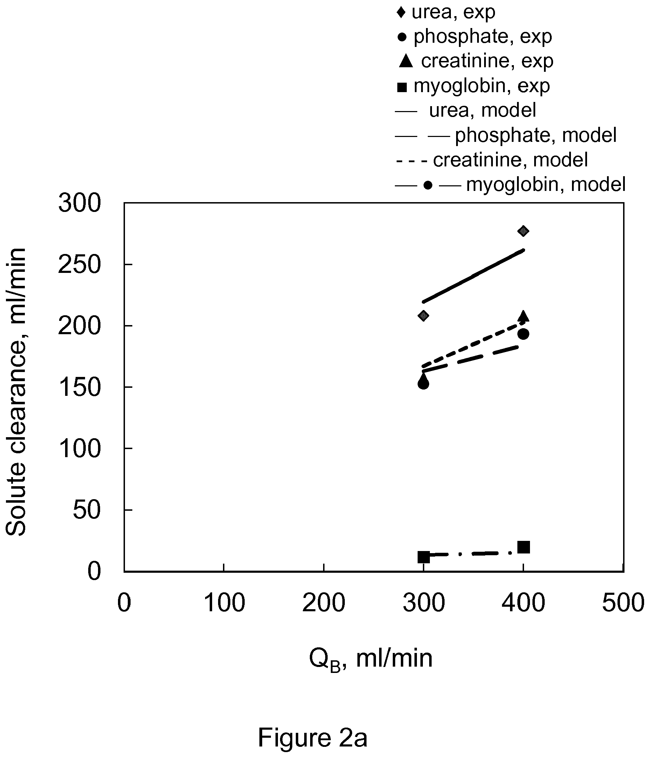

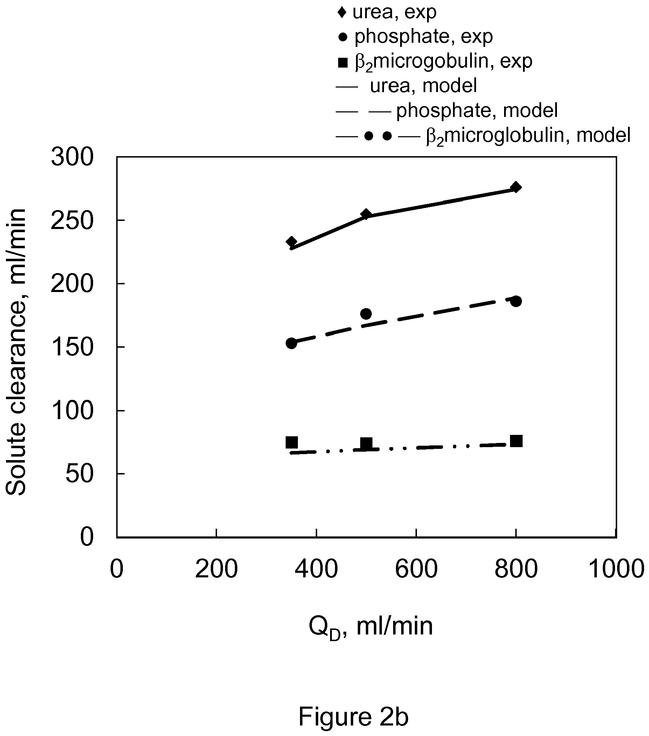

Figures 2a-b show the comparison between model-predicted and in vivo solute clearances obtained with FX CorDiax 80 (Fresenius Medical Care, Bad Homburg, Germany) (Figure 2a), and with Revaclear Max dialyzer (Gambro Dialysatoren GmbH, Germany, a subsidiary of Baxter International Inc.) (Figure 2b), at increasing blood and dialysate flow rates, respectively.

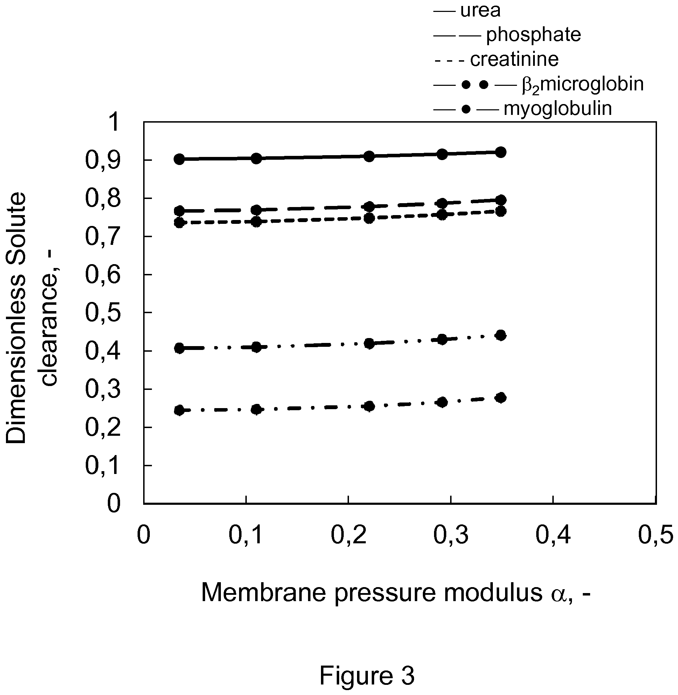

Figure 3 shows the effect of membrane pressure modulus, $\alpha$, at $Q_{UF}$ = 10 ml/min, L/$d_B$=1150, PD=0.65, $Q_D/Q_B$ = 1, on dimensionless solute clearances. Model parameters are as shown in Table 2.

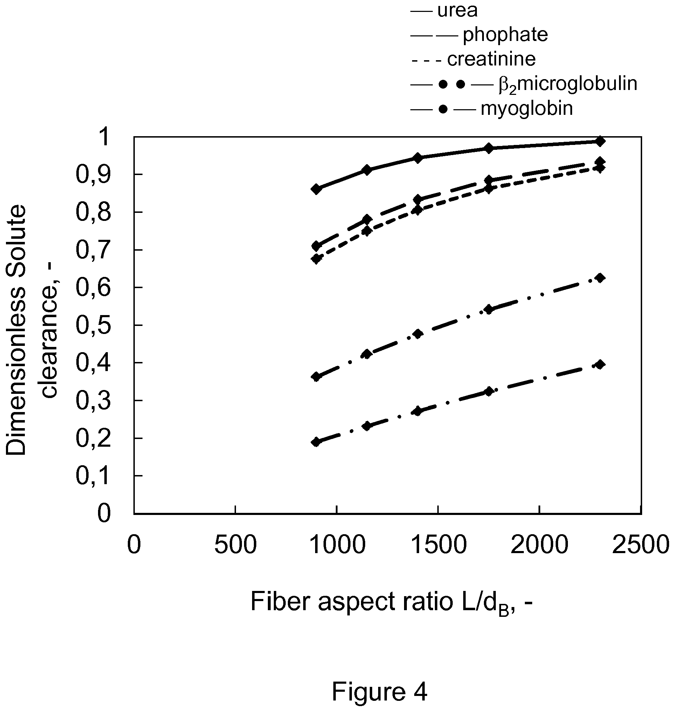

Figure 4 shows the effect of fiber aspect ratio, L/$d_B$, at $Q_{UF}$ = 10 ml/min, $\alpha$ = 0.22, PD=0.65, $Q_D/Q_B$ = 1, on dimensionless solute clearances. Model parameters are as shown in Table 2.

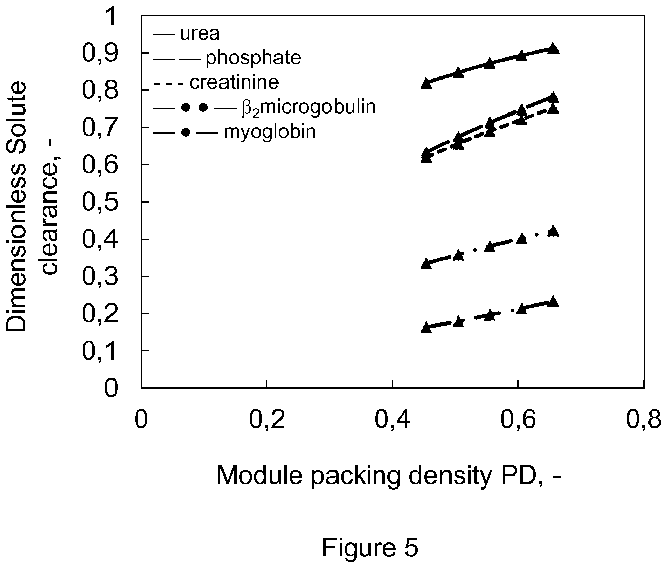

Figure 5 shows the effect of module packing density, PD, at $Q_{UF}$ = 10 ml/min, $Q_D/Q_B$ = 1, on dimensionless solute clearances at $\alpha$ = 0.22 and L/$d_B$= 1150. Model parameters are as shown in Table 2.

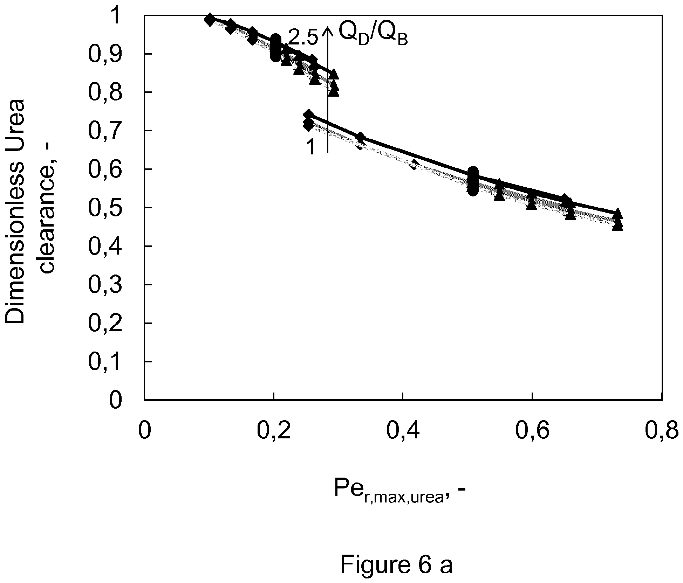

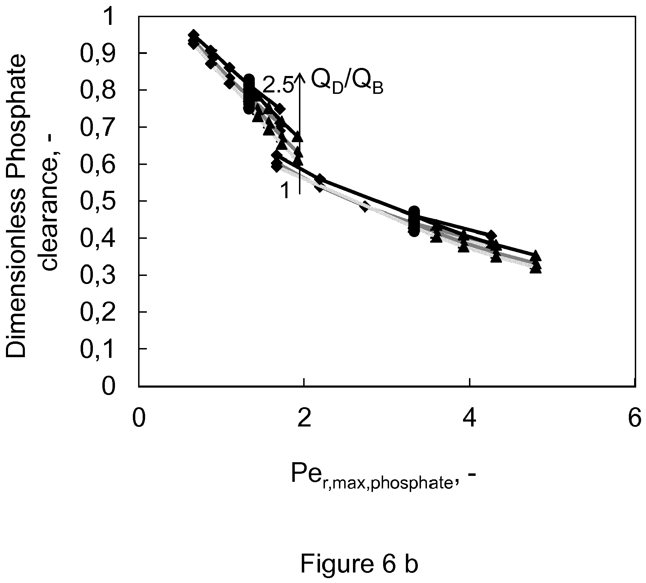

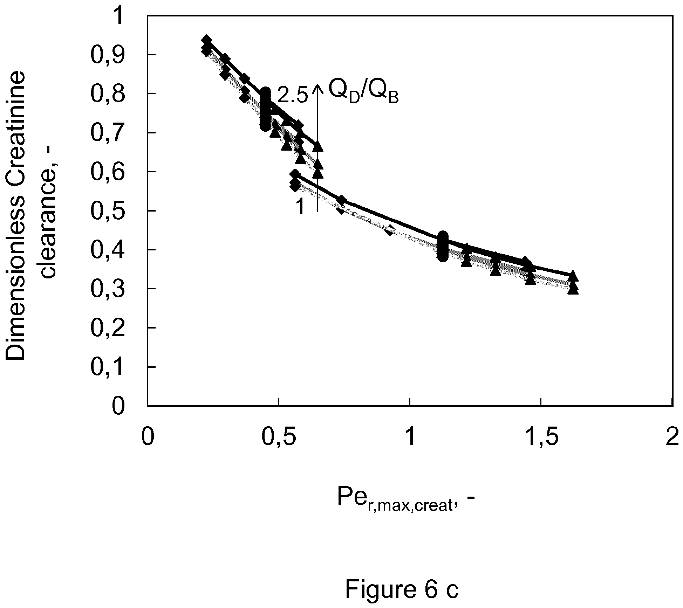

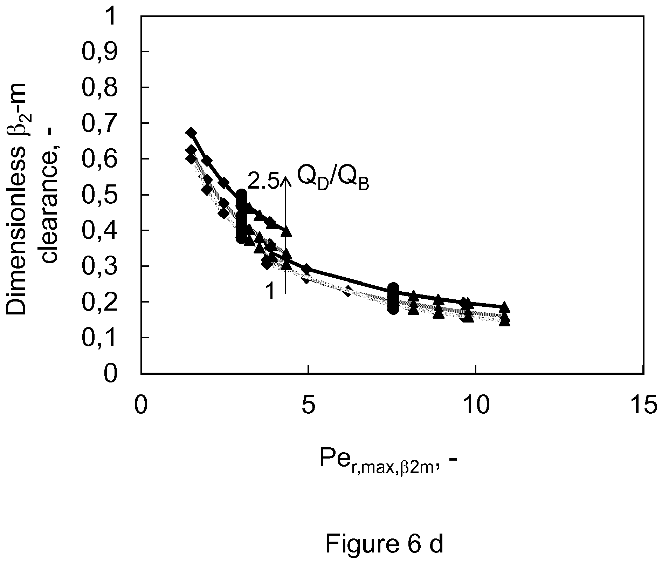

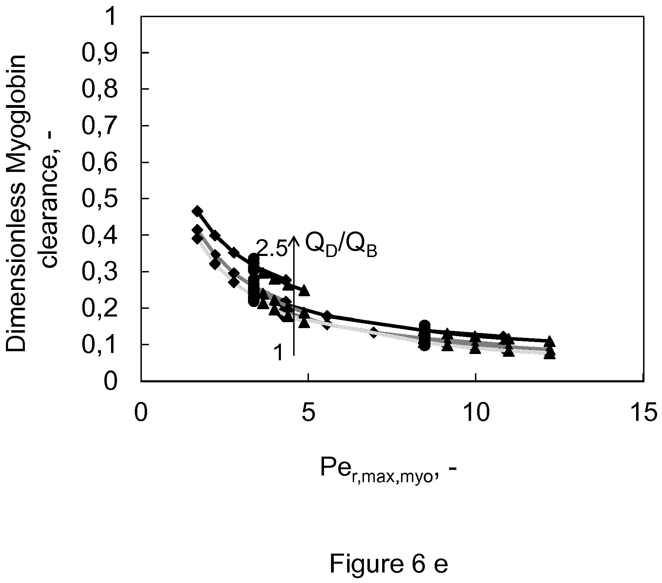

Figures 6a-e show the effect of maximal reduced Peclet numbers, $Pe_{r,max,j}$ at fixed dialysate-to-blood flow rate ratio, $Q_D/Q_B$, on dimensionless clearance of urea (in Figure 6a), phosphate (in Figure 6b), creatinine (in Figure 6c), $\beta$2-microglobulin (in Figure 6d), myoglobin (in Figure 6e). ● L/$d_B$=1150, PD=0.65; ▲ $\alpha$ = 0.22, L/$d_B$=1150; ♦ $\alpha$ = 0.22, PD=0.65. The shade of grey of the symbols identifies the extent of the net ultrafiltration flow rate: $Q_{UF}$ = 0 ml/min, - $Q_{UF}$ = 10 ml/min; - $Q_{UF}$ = 30 ml/min. Other parameters are as shown in Table 2.

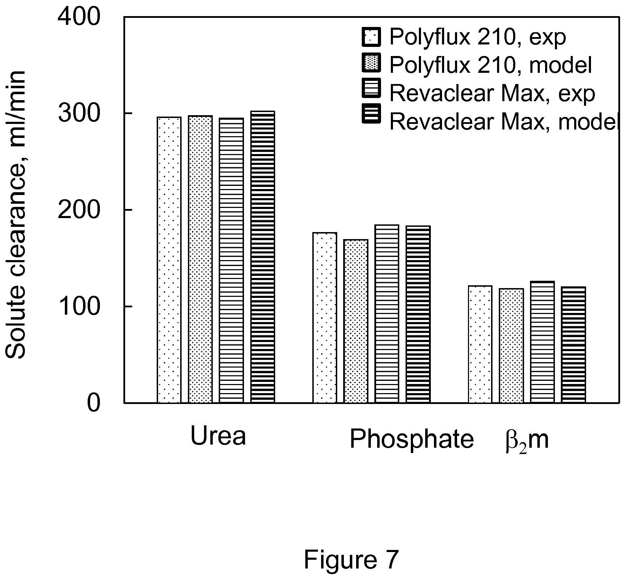

Figure 7 shows the model-predicted vs. experimental clearances of urea, phosphate and $\beta$2-microglobulin obtained with Gambro modules Polyflux 210H and Revaclear Max. Model parameters as disclosed by Ward and Ouseph, in Modification of membrane characteristics allows a reduction in dialyzer membrane area without loss of performance, J. Am. Soc. Nephrol. 18 (2007).

## Nomenclature

| | |
|---|---|
| $A_{tot} = \pi d_B L N$ | total membrane surface area [m$^2$] |
| $C_{i,j}$ | molar concentration of the j-th solute in i-th compartment [mol/m$^3$] |
| $Cl_j$ | clearance of j-th solute [m$^3$/s] |
| Cp | protein concentration [g/l] |
| $d_B = 2R_i$ | fiber inner diameter [m] |
| $d_D = d_B + 2\delta_m + 2\delta_D$ | outer diameter of dialysate compartment [m] |
| $d_H$ | housing diameter [m] |
| $D_{i,j}$ | j-th solute diffusivity in i-th compartment [m$^2$/s] |
| $H_{in}$ | inlet hematocrit [-] |
| $kc_p$ | protein mass transfer coefficient [m/s] |
| $k_m$ | membrane Darcy permeability [m$^2$] |
| $K_{UF}$ | ultrafiltration coefficient [m$^3$/(s*Pa)] |
| L | effective module length [m] |
| $L_p$ | membrane hydraulic permeability [m$^2$s/kg] |
| MW | molecular weight [kg/mol] |
| N | number of fibers [-] |
| $P_i$ | pressure in the i-th compartment [Pa] |
| PD | module packing density [-] |
| $Pe_{r,max,j}$ | maximal reduced Peclet number for the j-th solute [-] |
| $Q_i$ | fluid flow rate in i-th compartment [m$^3$/s] |
| $Q_{UF}$ | ultrafiltration rate [m$^3$/s] |
| r | radial coordinate [m] |
| $R_i$ | fiber inner radius [m] |
| $R_o = R_i + \delta_m$ | fiber outer radius [m] |
| $Re_B$ | Reynolds number in blood compartment [-] |
| $S_j$ | sieving coefficient of j-th solute [-] |
| $u_i$ | axial velocity in i-th compartment [m/s] |
| $u_{in,B} = 4Q_B / (N\pi d_B^2)$ | average axial velocity at blood-side entrance [m/s] |
| $v_i$ | radial velocity in i-th compartment [m/s] |
| z | axial coordinate [m] |

**Superscripts and subscripts**

| | |
|---|---|
| * | dimensionless |
| B | blood |
| $\beta 2m$ | b2-microglobulin |
| creat | creatinine |
| D | dialysate |
| i | i-th compartment |
| in | inlet |
| j | j-th solute |
| m | membrane |
| myo | myoglobin |
| p | protein |
| pl | plasma |
| out | outlet |
| RBC | red blood cells |
| urea | urea |
| w | wall |

**Greek symbols**

| | |
|---|---|
| $\alpha$ | pressure modulus [-] |
| $\delta_i$ | thickness of i-th compartment [m] |
| $\varepsilon_m$ | membrane porosity [-] |
| $\mu_i$ | fluid viscosity in i-th compartment [Pa s] |
| $\mu_{B,in}=\mu_{pl}(1+2.5H_{in})$ | reference viscosity in blood compartment [Pa s] |
| $\pi$ | oncotic pressure [Pa] |
| $\rho_{B,in}=\rho_{pl}(1-H_{in})+\rho_{RBC}H_{in}$ | reference density in blood compartment [kg/m$^3$] |
| $\rho_i$ | fluid density in i-th compartment [kg/m$^3$] |

**Detailed Description**

The present invention is based on analysing the complete set of dimensionless groups determining solute transport in hollow fiber dialyzers and their effect on solute clearance, and provides for a design of hollow fiber dialyzers for maximization of solute clearances. The design approach of dialyzers discussed herein accounts for the effect of all the dimensionless groups determining solute transport in all the compartments of the dialyzer and indicates that solute clearances may be generally maximized by reducing their maximal reduced Peclet number for constant dialysate-to-blood flow rate ratios and by increasing the pressure modulus for given membrane morphology. Based on that, the present invention provides, for the first time, for a defined combination of ranges for standard physical parameters required for such dialyzer. Experimental literature results show that this approach is applicable for the removal of small and middle molecules. The dialyzer design disclosed herein is useful for dialyzer scale-up in order to reduce the blood contact area and the blood-side volume, as well as the amount of medical waste, while ensuring adequate to improved solute removal for blood purification.

During hemodialysis, low molecular weight water soluble solutes (such as urea) are mostly removed by diffusion, whereas the removal of middle molecular weight solutes (such as $\beta 2$-microglobulin and myoglobin) generally require the superimposition of convective transport over pure diffusion by means of ultrafiltration and hemofiltration. Thus, the efficiency of hollow fiber dialyzers depends on their ability of combining diffusive and convective transport to maximize solute clearance. As stated above, solute transport in dialyzers is in particular strongly affected by dialyzer geometry, membrane properties and operating conditions.

Dialyzers generally comprise a cylindrical housing or casing. Located within the interior of the casing is a fiber bundle. Typically, the fiber bundle is comprised of a number of hollow fiber membranes that are oriented parallel to each other. The fiber bundle is encapsulated at each end of the dialyzer in a potting material to prevent blood flow around the fibers and to provide for a first flow space surrounding the membranes on the outside and a second flow space formed by the fiber cavities and the flow space above and below said potting material which is in flow communication with said fiber cavities. The dialyzers generally further consist of end caps capping the mouths of the tubular section of the device which also contains the fiber bundle. The dialyzer body also includes a dialysate inlet and a dialysate outlet. According to one embodiment of the invention, the dialysate inlet and dialysate outlet define fluid flow channels that are in a radial direction, i.e., perpendicular to the fluid flow path of the blood. The dialysate inlet and dialysate outlet are designed to allow dialysate to flow into an interior of the dialyzer, bathing the exterior surfaces of the fibers and the fiber bundle, and then to leave the dialyzer through the outlet. The membranes are designed to allow blood to flow therethrough in one direction with dialysate flowing on the outside of the membranes in opposite direction. Waste products are removed from the blood through the membranes into the dialysate. Accordingly, dialyzers typically include a blood inlet and a blood outlet, the blood inlet being designed to cause blood to enter the fiber membranes and flow therethrough. Dialysate is designed to flow through an inlet of the dialyzer and out of the dialyzer through an outlet, thereby passing the outside or exterior walls of the hollow fiber membranes.

A broad variety of parameters influence the performance of the device. Apart from the composition and pore structure of the membrane, the dialyzers' performance depends strongly on the geometry of the housing and the fiber bundle located therein, including the geometry of the single hollow fibers. Relevant parameters as concerns the fibers are the total membrane surface area ($A_{tot}$), the effective (accessible) length (L) of the fibers, the inner diameter ($d_B$) of the fibers, the wall thickness ($\delta_m$) of the fibers and their overall three-dimensional geometry. As mentioned before, the performance of dialyzers is also related to the membrane packing density which in turn is closely connected to the flow characteristics. A high membrane packing density increases the performance of the device as long as the uniformity of the flow is not affected. However, how the exact ranges for each of these parameters are and, on top of that, their interplay and optimal combination looks like, has so far not been determined and/or described.

The inventors have set out to determine said parameter ranges and their interplay based on known models and by using a a two-dimensional mathematical transport model aimed to (i) identify the most relevant dialyzer-related factors determining dialyzer efficiency and (ii) investigate how their interplay influences solute clearance in order to optimize dialyzer design. Motion, Darcy-Brinkman and Navier-Stokes equations were used to describe momentum transport in all the compartments of the dialyzer (i.e. blood-side, membrane, dialysate-side), respectively, whereas the convection-diffusion equation was used to describe the transport of low and middle molecular weight solutes in each dialyzer compartment. Non-Newtonian blood behaviour and effect of concentration polarization were also taken into account. Dimensional analysis was used to identify the dimensionless groups determining dialyzer efficiency and their effect on solute clearance was investigated by solving model equations under working conditions typical in clinical practice. A reference framework based on model predictions is proposed to help develop dialyzers allowing for the maximization of solute clearances in a cost-effective way.

For the model used to arrive at the dialyzer design claimed here, it was assumed that the fibers are evenly spaced and arranged in regular hexagonal arrays, and each fiber is surrounded by a uniform annulus. The interstitial spaces among neighbouring annuli are neglected. The solutes considered were urea (molecular weight MW 60Da), creatinine (molecular weight MW 113Da), phosphate (molecular weight MW 93Da), $\beta_2$-microglobulin (MW 11kDa) and myoglobin (MW 16.7kDa). With reference to the dialyzer scheme of Figure 1, the model assumptions are listed as follows: i. axial symmetry; ii. flow in the blood and dialysate compartment is steady-state and laminar; iii. isothermal conditions (T = 37 °C); iv. transport of momentum and mass occurs in 2D; v. incompressible fluids; vi. non-Newtonian behaviour of blood described according to Quemada et al. in General features of blood circulation in narrow vessels, in C.M. Rokkiewcz (Ed.), Artieries and arterial blood flow:biological and physiological aspects, Wien, Springer-Verlag 1983; vii. Newtonian behaviour of dialysate; viii. negligible effect of gravity; ix. the momentum transport in the blood and dialysate compartments was is described with the equation of motion and the Navier-Stokes equations, respectively (Bird et al.Transport phenomena, New York: J Wiley & Sons, inc., 2002); x. isotropic porous membrane; xi. momentum transport in the membrane can be described using a pseudo-homogeneous approach with the Darcy-Brinkman equation (Brinkman, A calculation of the viscos force exerted by a flowing fluid on a dense swarm of particles, Appl. Sci. Res. A(1) (1947)27-34); xii. creeping flow in membrane (Eloot et al. Optimisation of solute transport in dialysers using a three-dimensional finite volume model, Comp. Meth, Biomed. Eng. 9 (2006)363-370); xiii. exposition of all membranes to the same boundary conditions, irrespective of their position in the bundle; xiv. strongly diluted solutes; xv. constant sieving coefficients; xvi. the effect of concentration polarization can be described with the film theory model (Zydney, Bulk mass transport limitations during high flux hemodialysis, Artif. Org. 17(1993) 919-924).

A variety of general dialyzer designs can be utilized for accomplishing the present invention. According to one embodiment the hemodialyzers of the invention have an overall design such as those set forth in WO 2013/190022 A1. However, other designs can also be utilized without compromising the gist of the present invention.

Upon introducing the following non-dimensional coordinates and variables **(1)**:

$$z^* = \frac{z}{L} \; ; \; r^* = \frac{2r}{d_B} \; ; \; u_i^* = \frac{u_i}{u_{B,in}} \; ; \; v_i^* = \frac{2v_i}{u_{B,in} d_B / L} \; ; \; P_i^* = \frac{4P_i}{u_{B,in} \mu_{B,in} L / d_B^2} \; ; \; C_{i,j}^* = \frac{C_{i,j}}{C_{B,in,j}} \; ;$$

$$\rho_B^* = \frac{\rho_B}{\rho_{B,in}} \; ; \; \mu_B^* = \frac{\mu_B}{\mu_{B,in}} \; ; \; \pi^* = \frac{\pi}{\pi_0} \; ; \; C_p^* = \frac{C_p}{C_{P,in}}$$

(1)

the governing conservation equations may be re-arranged in dimensionless form to give:

Continuity equation **(2)** (i = B, m, D)

$$\frac{1}{r^*} \frac{\partial}{\partial r^*} \left( r^* v_i^* \right) + \frac{\partial u_i^*}{\partial z^*} = 0$$

(2)

Blood side (B) - momentum balance equations **(3)** and **(4)** :

$$r) \quad \frac{u_{B,in} d_B \rho_{B,in}}{2\mu_{B,in}} \frac{d_B}{2L} \rho_B^* \left( v_B^* \frac{\partial v_B^*}{\partial r^*} + u_B^* \frac{\partial v_B^*}{\partial z^*} \right) = -\left(\frac{2L}{d_B}\right)^2 \frac{\partial P_B^*}{\partial r^*} + \mu_B^* \frac{\partial}{\partial r^*} \left( \frac{1}{r^*} \frac{\partial}{\partial r^*} \left( r^* v_B^* \right) \right) + \left(\frac{d_B}{2L}\right)^2 \frac{\partial^2 v_B^*}{\partial z^{*2}}$$

(3)

$$z) \quad \frac{u_{B,in} d_B \rho_{B,in}}{2\mu_{B,in}} \frac{d_B}{2L} \rho_B^* \left( v_B^* \frac{\partial u_B^*}{\partial r^*} + u_B^* \frac{\partial u_B^*}{\partial z^*} \right) = -\frac{\partial P_B^*}{\partial z^*} + \mu_B^* \left( \frac{1}{r^*} \frac{\partial}{\partial r^*} \left( r^* \frac{\partial u_B^*}{\partial r^*} \right) \right) + \left(\frac{d_B}{2L}\right)^2 \frac{\partial^2 u_B^*}{\partial z^{*2}}$$

(4)

Membrane wall (m) - momentum balance equations **(5)** and **(6)**:

$$z) \quad -\frac{k_m}{(d_B/2)^2} \frac{\mu_{B,in}}{\mu_m} \frac{\partial P_m^*}{\partial z^*} + \frac{1}{\varepsilon_m} \frac{k_m}{(d_B/2)^2} \left[ \frac{1}{r^*} \frac{\partial}{\partial r^*} \left( r^* \frac{\partial u_m^*}{\partial r^*} \right) + \left(\frac{d_B}{2L}\right)^2 \frac{\partial^2 u_m^*}{\partial z^{*2}} \right] - u_m^* = 0$$

(5)

$$r) \quad -\frac{k_m L^2}{\delta_m (d_B/2)^3} \frac{2\delta_m}{d_B} \frac{\mu_{B,in}}{\mu_m} \frac{\partial P_m^*}{\partial r^*} + \frac{1}{\varepsilon_m} \frac{k_m}{(d_B/2)^2} \frac{\partial}{\partial r^*} \left( \frac{1}{r^*} \frac{\partial}{\partial r^*} \left( r^* v_m^* \right) \right) - v_m^* = 0$$

(6)

Dialysate side (D) - momentum balance equations **(7)** and **(8)**:

$$r) \cdot \frac{u_{B,in} d_B \rho_{B,in}}{2\mu_{B,in}} \frac{d_B}{2L} \frac{\rho_D}{\rho_{B,in}} \frac{\mu_{B,in}}{\mu_D} \left( v_D^* \frac{\partial v_D^*}{\partial r^*} + u_D^* \frac{\partial v_D^*}{\partial z^*} \right) = -\left(\frac{2L}{d_B}\right)^2 \frac{\partial P_D^*}{\partial r^*} + \frac{\partial}{\partial r^*} \left( \frac{1}{r^*} \frac{\partial}{\partial r^*} \left( r^* v_D^* \right) \right) + \left(\frac{d_B}{2L}\right)^2 \frac{\partial^2 v_D^*}{\partial z^{*2}}$$

(7)

$$z) \cdot \frac{u_{B,in} d_B \rho_{B,in}}{2\mu_{B,in}} \frac{d_B}{2L} \frac{\rho_D}{\rho_{B,in}} \frac{\mu_{B,in}}{\mu_D} \left( v_D^* \frac{\partial u_D^*}{\partial r^*} + u_D^* \frac{\partial u_D^*}{\partial z^*} \right) = -\frac{\partial P_D^*}{\partial z^*} + \left( \frac{1}{r^*} \frac{\partial}{\partial r^*} \left( r^* \frac{\partial u_D^*}{\partial r^*} \right) \right) + \left(\frac{d_B}{2L}\right)^2 \frac{\partial^2 u_D^*}{\partial z^{*2}}$$

(8)

Equations 1-8 may be solved with the following boundary conditions: fiber axis and dialysate-side outer boundary impervious to momentum and fluid (i.e. $\forall z^*$ at $r^* = 0$ and $r^* = d_D/d_B$, $\partial u_i^*/\partial r^* = 0$ and $v_i^* = 0$, i = B, D); fully developed velocity profile at the blood and dialysate entrances; continuity of axial and radial velocity at blood-membrane and membrane-dialysate interfaces; continuity in pressure at membrane-dialysate interface; interfaces at the end sections of the membrane impervious to momentum and fluid (i.e. at $d_B/2 < r^* < 2R_0/d_B$, at $z^* = 0$ and $z^* = 1$, $u_m^* = 0$); constant pressure at blood and dialysate-sides exits. The oncotic pressure exerted by the plasma proteins, assumed to be completely rejected by the membrane, was implemented as a discontinuous pressure drop at the interface between the blood compartment and membrane wall (i.e. $\forall z^*$ at $r^* = 1$, $P_m^* = P_B^* - \pi_0^*\pi^*$), and calculated as equation **(9)** :

$$\pi^* = Cp_{in}\frac{\pi_1}{\pi_0}Cp_w^* + Cp_{in}^2\frac{\pi_2}{\pi_0}Cp_w^{*2} + Cp_{in}^3\frac{\pi_3}{\pi_0}Cp_w^{*3} \tag{9}$$

where $\pi_1 = 0.21$ mmHg l/g, $\pi_2 = 1.6 \times 10^{-3}$ mmHg l$^2$/g$^2$, $\pi_3 = 9 \times 10^{-6}$ mmHg l$^3$/g$^3$. The protein concentration at the blood-membrane skin interface was computed according to the film theory model as **(10)**:

$$Cp_w^* = Cp^* \exp(v_{B|_{r=dB/2}}/kc_p) \tag{10}$$

The concentration of the completely rejected protein varies with the local flow rate as (11):

$$\frac{d(Q_B(z)/Q_B C_p^*)}{dz^*} = 0 \tag{11}$$

with $Cp^* = 1$ at $z^* = 0$. The protein mass transfer coefficient, which varies with the axial coordinate, was computed from the Sherwood number, $Sh_p$, as (12):

$$Sh_p = 1.62\left(2\,Re_B\,Sc_{B,p}\,\frac{d_B}{z}\right)^{1/3} = \frac{kc_p d_B}{D_{B,p}} \tag{12}$$

where (13) :

$$Sc_B = \frac{\mu_{B,in}}{\rho_{B,in}D_{B,p}} \tag{13}$$

In Equation 3 and 4, blood viscosity was assumed to vary in both radial and axial direction according to Quemada General features of blood circulation in narrow vessels, in: C.M. Rokkiewcz (Ed.), Arteries and arterial blood flow: biological and physiological aspects, Wien, Springer-Verlag,1983.

Mass conservation for a j-th solute (j = urea, creatinine, phosphate, $\beta$2-microglobulin, myoglobin):

Blood side - convection-diffusion equation **(14)**:

$$\frac{u_{B,in}L}{D_{B,j}}\left(\frac{d_B}{2L}\right)^2\left(v_B^*\frac{\partial C_{B,j}^*}{\partial r^*} + u_B^*\frac{\partial C_{B,j}^*}{\partial z^*}\right) = \frac{1}{r^*}\frac{\partial}{\partial r^*}\left(r^*\frac{\partial C_{B,j}^*}{\partial r^*}\right) + \left(\frac{d_B}{2L}\right)^2\frac{\partial^2 C_{B,j}^*}{\partial z^{*2}} \tag{14}$$

Membrane - convection-diffusion equation **(15)**:

$$\frac{u_{B,in}L}{D_{B,j}}\left(\frac{d_B}{2L}\right)^2\frac{D_{B,j}}{D_{m,j}}S_j\left(v_m^*\frac{\partial C_{m,j}^*}{\partial r^*} + u_m^*\frac{\partial C_{m,j}^*}{\partial z^*}\right) = \frac{1}{r^*}\frac{\partial}{\partial r^*}\left(r^*\frac{\partial C_{m,j}^*}{\partial r^*}\right) + \left(\frac{d_B}{2L}\right)^2\frac{\partial^2 C_{m,j}^*}{\partial z^{*2}} \tag{15}$$

Dialysate side - convection-diffusion equation **(16)**:

$$\frac{u_{B,in}L}{D_{B,j}}\left(\frac{d_B}{2L}\right)^2 \frac{D_{B,j}}{D_{D,j}}\left(v_D^* \frac{\partial C_{D,j}^*}{\partial r^*}+u_D^* \frac{\partial C_{D,j}^*}{\partial z^*}\right)=\frac{1}{r^*}\frac{\partial}{\partial r^*}\left(r^* \frac{\partial C_{D,j}^*}{\partial r^*}\right)+\left(\frac{d_B}{2L}\right)^2 \frac{\partial^2 C_{D,j}^*}{\partial z^{*2}} \tag{16}$$

Equations 14-16 were solved with the following boundary conditions: uniform and zero solute concentration profile in the stream entering the blood and the dialysate compartments, respectively; fiber axis and dialysate-side outer boundary impervious to mass (i.e. $\forall z^*$ at $r^* = 0$ and $r^* = d_D/d_B$, $\partial C_{i,j}^*/\partial r^* = 0$, $i = B, D$) ; continuous solute concentrations and mass fluxes at the interfaces of neighbouring regions; interfaces at the end sections of the membrane impervious to mass (i.e. at $d_B/2 < r^* < R_o$, at $z^* = 0$ and $z^* = 1$, $\partial C_{m,j}^*/\partial z^* = 0$) ; solute concentrations in the streams leaving blood and dialysate compartments do not change any further (i.e. at $0 < r^* < d_B/2$, $z^* = 1$ and at $R_o < r^* < d_D/2$, $z^* = 0$, $\partial C_{i,j}^*/\partial z^* = 0$, $i = B, D$).

Analysis of Equations 1-16 and related boundary conditions shows that dialyzer efficiency is determined by the values of the independent dimensionless groups reported in Table 1.

**Table 1**: Dimensionless groups determining dialyzer performance

| Dimensionless group | Description |
|---|---|
| 1. $u_{in,B}d_B\rho_{B,\,in}/\,(2\mu_{B,\,in}) = Re_B$ | Reynolds number at blood-side inlet |
| 2. $L/d_B$ | fiber aspect ratio |
| 3. $(128k_mL^2/(d_B{}^3\delta_m))^{1/2}= \alpha$ | membrane pressure modulus |
| 4. $2\delta_m/d_B$ | membrane thickness-to-fiber inner radius ratio |
| 5. $\mu_{B,in}/\mu_m$ | blood-to-membrane side viscosity ratio |
| 6. $\mu_{Bin}/\mu_D$ | blood-to-dialysate side viscosity ratio |
| 7. $\rho_D/\rho_{B,in}$ | dialysate-to-blood density ratio |
| 8. $2\delta_D/d_B$ | dialysate side thickness-to-fiber inner radius ratio |
| 9. $Q_D/Q_B$ | dialysate-to-blood side inlet flow rate ratio |
| 10. $4\pi_0 d_B{}^2/(2\mu_{B,in}u_{in,B}L)$ | dimensionless oncotic pressure |
| 11. $u_{in,B}L/D_{B,j}\,(d_B/(2L))^2 = Pe_{r,max,\,j}$ | maximal reduced Peclet number of the j-th solute |
| 12. $D_{B,j}/D_{m,j}$ | blood-to-membrane j-th solute diffusivity ratio |
| 13. $D_{B,j}/D_{D,j}$ | blood-to-dialysate j-th solute diffusivity ratio |
| 14. $2kc_p/(u_{in,B}d_B/L)$ | dimensionless mass transfer coefficient at B-m interface |

Such dimensionless groups account for module geometry, properties of the fluids, membrane properties, operating conditions and solute properties. The physical meaning of most of them has previously been reported in literature, e.g. by R.B. Bird et al in Transport phenomena, New York: J. Wiley & Sons, Inc., 2002, by R.L. Fournier, in Basic Transport Phenomena in Biomedical Engineering, 3rd ed., CRC Press, Boca Raton, 2012, and by A. Apelblat et al in A mathematical analysis of capillary tissue fluid exchange, Biorheology 11 (1974) 1-49. In particular, the value of $\alpha$, which accounts for the ability of the membrane to enable ultrafiltration for given pressure drops in the blood and dialysate compartments, was kept in the range reported for low and high flux ultrafiltration membranes, typically adopted in hemodialysis. In particular, in order to take into account for the fiber curvature, the membrane Darcy permeability was calculated from the hydraulic permeability as **(17)**:

$$k_m = L_p \frac{\mu_m}{\varepsilon_m}\frac{d_B}{2}\log\frac{\frac{d_B}{2}+\delta_m}{\frac{d_B}{2}} \tag{17}$$

wherein (18):

$$L_p = \frac{K_{UF}}{A_{tot}} \qquad (18)$$

The product between the j-th maximal axial Peclet number at the blood-side inlet, $Pe_{ax,B,max,j} = U_{in,B}L/D_{B,j}$, and the squared membrane shape ratio, $d_B/(2L)$, is referred to as the maximal reduced Peclet number, $Pe_{r,max,j}$, and provides a measure of the relative importance of convective-to-diffusive solute transport along the radial coordinate at a given membrane pressure modulus. Finally, the effect of the membrane thickness-to-fiber inner radius ratio, $2\delta_m/d_B$, and the dialysate-side thickness-to-fiber inner radius ratio, $2\delta_D/d_B$, was better evaluated in terms of the module packing density, defined as (19) :

$$PD = \frac{4N(d_B/2 + \delta_m)^2}{d_h^2} = \left( \frac{1 + \dfrac{2\delta_m}{d_B}}{1 + \dfrac{2\delta_m}{d_B} + \dfrac{2\delta_D}{d_B}} \right)^2 = f(\delta_D/d_B, \delta_m/d_B) \qquad (19)$$

The dialyzer efficiency was assessed in terms of dimensionless solute clearances, calculated as **(20)**:

$$Cl_j^* = \frac{C_{B,in,j} - C_{B,out,j}}{C_{B,in,j}} + S_j \frac{Q_{UF}}{Q_B} \qquad (20)$$

The model was validated by comparing model-predicted clearances of each solute to corresponding experimental data reported in literature obtained by operating dialyzer modules at increasing blood and dialysate flow rates. Due to the lack of comprehensive data sets of solute diffusivities for each dialyzer membrane, solute diffusivities in the membrane were found iteratively until model-predicted solute clearances matched the experimental data, assuming blood and dialysate-sides diffusivities reported in Table 2.

**Table 2**: Model parameter values used for model predictions

| Parameter | Value | unit |
|---|---|---|
| L | 23 | cm |
| $d_B$ | 200 | $\mu$m |
| $\delta_m$ | 35 | $\mu$m |
| $d_H$ | 38 | mm |
| N | 12960 | - |
| $H_{in}$ | 0.3 | - |
| $\mu_{pl}$ | $1.3 \times 10^{-3}$ | Pa s |
| $C_{p,\,in}$ | 58 | g/l |
| $\rho_D$ | 1008 | kg/m$^{-3}$ |
| $\mu_D$ | $0.687 \times 10^{-3}$ | Pa s |
| $K_{UF}$ | 48 | ml/h/mmHg |
| $\varepsilon_m$ | 0.8 | - |
| $P_{B,out}$ | 0.1 | bar |
| $Q_{UF}$ | 0-30 | ml/min |
| $Q_B$ | 200-500 | ml/min |
| $Q_D$ | 500 | ml/min |

(continued)

| Parameter | Value | unit |
|---|---|---|
| $D_{B,j}$ | 17.5 x $10^{-10}$ (urea), 2.57 x $10^{-10}$ (phosphate), 7.9 x $10^{-10}$ (creat), 1.18 x $10^{-10}$ ($\beta_2$m), 1.05 x $10^{-10}$ (myo) | m²/s |
| $D_{m,j}$ | 1.9 x $10^{-10}$ (urea), 2.5 x $10^{-10}$ (phosphate), 9.5 x $10^{-11}$ (creat), 5.9 x $10^{-11}$ ($\beta_2$m), 3.75 x $10^{-11}$ (myo) | m²/s |
| $D_{D,j}$ | 19 x $10^{-10}$ (urea), 2.67 x $10^{-10}$ (phosphate), 9.66 x $10^{-10}$ (creat), 2.09 | m²/s |
|  | x $10^{-10}$ ($\beta_2$m), 1.85 x $10^{-10}$ (myo) | |
| $S_j$ | 1 (j=urea, creat, phosphate, $\beta_2$m); 0.9 (j=myo) | |

In accordance with the available experimental results used for model validation, solute clearances were calculated from the
dialysate flow rate as (21):

$$Cl_j = (Q_D + Q_{UF})\frac{C_{D,out,j}}{C_{pl,in,j}}$$

(21)

The dialyzer design derived from the above calculations and as described herein take into account how geometric, transport and operating parameters affect solute clearance, and provide a framework that permits the adjustment of such parameters in order to maximize solute clearances, so that newly designed dialyzers may be realized in a cost-effective way. In fact, no model reported in literature was able to perform an in-depth analysis of all the dimensionless groups determining solute clearance, nor to find design criteria for dialyzer optimization.

Dimensional analysis shows that a crucial dimensionless group to assess the enhancement of transport of a j-th solute (i.e. the solute clearances) is the maximal j-th reduced Peclet number, $Pe_{r,max,j}$. Figure 6 shows that, when the values of the dimensionless solute clearances are rearranged vs. $Pe_{r,max,j}$, all curves reported in Figures 3-5 converge towards one curve only for a constant value of $Q_D/Q_B$ and for a given $D_m/D_B$. This holds true for both lower and middle molecular weight solutes. This results suggests that the maximal reduced Peclet number accounts well for the effect of the most relevant dimensionless groups determining solute transport. This observation is also supported by results reported by Ward and Ouseph in Modification of membrane characteristics allows a reduction in dialyzer membrane area without loss of performance, J. Am. Soc. Nephrol. 18 (2007) on the clearance of urea, phosphate and β2-microglobulin determined experimentally with Polyflux 210H and Revaclear Max dialyzers operated at the same working conditions

(Figure 7). At the same operating conditions, the authors report an equivalent dialyzer performance for the two dialyzer modules over the wide range of solute molecular weights, despite their significantly different geometry. In particular, urea, phosphate and β2-microglobulin clearances have been reported to be equal to around 296, 169 and 121 ml/min, respectively, for Polyflux 210H ($A_{tot}$ = 2.1 m², $d_B$ = 215 $\mu$m, $\delta_m$ = 50 $\mu$m, PD = 0.45) and 295, 184 and 126 ml/min, respectively, for Revaclear Max ($A_{tot}$ = 1.8 m², $d_B$ = 190 $\mu$m, $\delta_m$ = 35 $\mu$m, PD = 0.56), both operated at $Q_B$ = 400 ml/min, $Q_D$ = 800 ml/min and $Q_{UF}$ = 0 ml/min. These conditions yield $Pe_{r,max,urea}$ = 0.43, $Pe_{r,max,PO4}$ - 2.93 and $Pe_{r,max,\beta2m}$ = 15.89 for Polyflux 210H and $Pe_{r,max,urea}$ - 0.43, $Pe_{r,max,PO4}$ = 2.92 and $Pe_{r,max,\beta2m}$ = 15.88 for Revaclear Max.

Figures 6a-e also show that dimensionless clearance of a given solute may be maximized by minimizing the value of its maximal reduced Peclet number for given $Q_B/Q_D$ and $D_m/D_B$. Furthermore, Figures 6a, 6b and 6c show that the net ultrafiltration flow rate has negligible influence on the dimensionless clearance of urea, phosphate and creatinine, respectively, whereas Figure 6d and 6e show that the effect of the net ultrafiltration rate increases for dimensionless β2-microglobulin and myoglobin clearance.

Figures 6a-e also evidence that the effect of the net ultrafiltration flow rate increases as the maximal reduced Peclet number increases.

Based on the above, the present inventors have found that with the inventive specific combination of geometric aspects of the membrane, 10-15 % increase of urea clearance, and about 20-100% increase of myoglobin clearance may be accomplished in comparison with prior art membranes.

According to the invention this and other objects are achieved, in full or at least in part, by a hemodialyzer for the purification of blood comprising a bundle of synthetic hollow fiber membranes having an inner diameter ($d_B$) within the range of 168-175 $\mu$m, and a fiber wall thickness ($\delta_m$) within the range of 25-26 $\mu$m, wherein the fiber membranes have an effective length (L) within the hemodialyzer within the range of 207-287 mm, and wherein the bundle of hollow fiber membranes has a packing density within the range of 57.5-60.0%, and wherein the total membrane surface area ($A_{tot}$) is in the range of from 1.64 to 1.73 m$^2$.

According to one embodiment of the invention, the optimized dialyzer is further defined by certain dimensionless parameters as described before, specifically by

(a) the membrane pressure modulus ($\alpha$), which may be in the range of from 0.31 to 0.40;
(b) the maximal reduced Peclet number for urea ($Pe_{r,max,\ urea}$) which may be in the range of from 0.45 to 0.49 (for a $Q_B$ of 500 ml/min and $D_b$=1.75e$^{-9}$ m$^2$/s); and
(c) the membrane-to-blood side solute diffusivity ratio, $D_m/D_B$, the value of which is determined by membrane morphology for any solute.

With the claimed specific geometric properties, the present inventors have found that increased clearance, as exemplified by urea clearance and myoglobin clearance, may be accomplished in comparison with prior art membranes dialyzers.

In one embodiment the hemodialyzer has a housing with an inner diameter ($d_H$) within the range of 31-35 mm.

In another embodiment the hemodialyzer membranes have a molecular retention onset (MWRO) of between 5 kDa and 20 kDa and a molecular weight cut-off (MWCO) of between 25 kDa and 320 kDa as determined by dextran sieving before blood contact to the membrane.

In another embodiment the hemodialyzer membranes have a molecular retention onset (MWRO) of between 5 kDa and 10 kDa and a molecular weight cut-off (MWCO) of between 25 kDa and 65 kDa as determined by dextran sieving before blood contact to the membrane.

In another embodiment the hemodialyzer membranes have a molecular retention onset (MWRO) of between 9 kDa and 14 kDa and a molecular weight cut-off (MWCO) of between 55 kDa and 130 kDa as determined by dextran sieving before blood contact to the membrane.

In another embodiment the hemodialyzer membranes have a molecular retention onset (MWRO) of between 15 kDa and 20 kDa and a molecular weight cut-off (MWCO) of between 170 kDa and 320 kDa as determined by dextran sieving before blood contact to the membrane.

In a further embodiment, the housing of the hemodialyzer has an inner total volume ($V_{tot}$) within the range of 195 to 199 ml.

In even a further embodiment, the fiber bundle within the hemodialyzer consists of 80-95 % of crimped fibers and of 5% to 15% non-crimped fibers, relative to the total number of fibers in the bundle.

In one embodiment the hollow fiber membranes within the hemodialyzer have an inner diameter ($d_B$) of 170-174 $\mu$m.

In another embodiment the hollow fiber membranes within the hemodialyzer have a fiber wall thickness ($\delta_m$) of 25 $\mu$m.

In a further embodiment, the bundle of hollow fiber membranes has a packing density of from 58.0% to 60.0% within the hemodialyzer.

The semipermeable hemodialysis membrane according to one embodiment of the invention comprises a copolymer of acrylonitrile and sodium methallyl sulfonate.

The semipermeable hemodialysis membrane according to another embodiment of the invention comprises at least one hydrophilic polymer and at least one hydrophobic polymer. In one embodiment, said at least one hydrophilic polymer and at least one hydrophobic polymer are present as coexisting domains on the surface of the dialysis membrane. According to one embodiment of the invention, the polymer solution may contain an additional hydrophobic polymer, such as, for example, polyamide, which is added to the polymer composition in low amounts.

The hydrophobic polymer may be chosen from the group consisting of poly(aryl)ethersulphone (PAES), polypropylene (PP), polysulphone (PSU), polycarbonate (PC), polyacrylonitrile (PAN), polyamide (PA), polytetrafluorethylene (PTFE), polyethersulphone (PES) or combinations thereof. In a specific embodiment of the invention, the hydrophobic polymer is chosen from the group consisting of poly(aryl)ethersulphone (PAES) and polysulphone (PSU). The hydrophilic polymer will be chosen from the group consisting of polyvinylpyrrolidone (PVP), polyethyleneglycol (PEG), polyvinylalcohol (PVA), a copolymer of polypropyleneoxide and polyethyleneoxide (PPO-PEO), or combinations thereof. In another embodiment of the invention, the hydrophilic polymer may be chosen from the group consisting of polyvinylpyrrolidone (PVP), polyethyleneglycol (PEG) and polyvinylalcohol (PVA). In one specific embodiment of the invention, the hydrophilic polymer is polyvinylpyrrolidone (PVP).

The dialysis membrane according to the invention may be a hollow fiber having an asymmetric foam- or sponge-like and/or a finger-like structure with a separation layer present in the innermost layer of the hollow fiber. Both membrane types and methods for producing same are known in the art.

The manufacturing of a hollow membrane follows a phase inversion process, wherein a polymer or a mixture of polymers is dissolved in a solvent to form a polymer solution. The solution is degassed and filtered before spinning.

The temperature of the polymer solution is adjusted during passage of the spinning nozzle (or slit nozzle) whose temperature can be regulated and is closely monitored. The polymer solution is extruded through said spinning nozzle (for hollow fibers) or a slit nozzle (for a flat film) and after passage through the so-called spinning shaft enters into said precipitation bath containing a non-solvent for the polymer and optionally also a solvent in a concentration of up to 20 wt.-%. To prepare a hollow fiber membrane, the polymer solution preferably is extruded through an outer ring slit of a nozzle having two concentric openings. Simultaneously, a center fluid is extruded through an inner opening of the spinning nozzle. At the outlet of the spinning nozzle, the center fluid comes into contact with the polymer solution and at this time the precipitation is initialized. The precipitation process is an exchange of the solvent from the polymer solution with the non-solvent of the center fluid. By means of this exchange the polymer solution inverses its phase from the fluid into a solid phase. In the solid phase the pore structure and the pore size distribution is generated by the kinetics of the solvent/non-solvent exchange. The process works at a certain temperature which influences the viscosity of the polymer solution. For preparing membranes according to the invention, the temperature of the spinning nozzle and, consequently, of the polymer solution and the center fluid as well as the temperature of the spinning shaft should be carefully controlled. In principle, membranes of the invention can be prepared at a comparatively broad temperature range.

The hollow fiber membranes according to the invention are prepared with a wall thickness of 25-26 $\mu$m. The membranes may in one embodiment be produced with a wall thickness of 25 $\mu$m for full efficiency in the context of the present invention.

The inner diameter of the hollow fiber membranes of the present invention are in range of from 168 $\mu$m to 175 $\mu$m, or in the range of 170-174, but may in one embodiment be produced with an inner diameter of 170 $\mu$m for full efficiency in the context of the present invention.

Further, the packing density of the hollow fiber membranes in the hemodialyzers is of importance for the mass transfer boundary layers that develop in the fluid adjacent to the membrane surface on the outer shell surface of the hollow fiber. The packing density is the sum of the cross-sectional area of all hollow fiber membranes present in the dialyzer and the percentage this amounts to of the cross-sectional area of the part of the dialyzer housing comprising the bundle of semi-permeable hollow fiber membranes. If N hollow fiber membranes are present in the bundle of semi-permeable hollow fiber membranes, $2R_o$ is the outer diameter of a single hollow fiber membrane, and $d_H$ is the inner diameter of the part of the dialyzer housing comprising the bundle, the packing density can be calculated according to $N*(2R_o/d_H)^2$.

According to the present invention, the packing density of the hollow fiber membranes in the hemodialyzer is within the range of 57,5-60.0%, 58-60%, or in the range of 59-60.0%. In one embodiment the packing density is 59.8%.

As stated above, also the geometry of the dialyzer housing as such affects the solute transfer, and according to one embodiment of the invention, the housing has an inner diameter ($d_H$) within the range of 31-35 mm, wherein $d_H$ is the inner diameter of the part of the dialyzer housing comprising the bundle.

The membrane may in one embodiment have a molecular retention onset (MWRO) of between 5 kDa and 20 kDa and a molecular weight cut-off (MWCO) of between 25 kDa and 320 kDa as determined by dextran sieving before blood contact to the membrane (see Example 2 below), and the membrane may be classified as a high-flux, a medium cut-off or a high cut-off membrane.

A typical fiber bundle with fibers according to the invention, wherein the fibers have a wall thickness of 25 $\mu$m and an inner diameter of 170 $\mu$m, and which is located within a housing having an inner diameter of, for example, 34 mm, wherein the fibers have a housing fiber length (L) of 212 mm and wherein packing densities of between 59 to 60.0% are realized, will contain about 14 600 fibers, providing for an effective total fiber length (L*N) of 3.107 km and an effective surface area of about 1.66 m$^2$. In another example, the fibers have a wall thickness of 25 $\mu$m and an inner diameter of 170 $\mu$m, and which is located within a housing having an inner diameter of, for example, 31 mm, wherein the fibers have a housing fiber length (L) of 260 mm and wherein packing densities of between 59 to 60.0% are realized, will contain about 12 000 fibers, also providing for an effective total fiber length (L*N) of 3.107 km and an effective surface area of about 1.66 m$^2$.

According to one aspect of the present invention, a bundle of hollow fiber membranes is present in the housing or casing, wherein the bundle comprises crimped fibers. The bundle may contain only crimped fibers, such as described, for example, in EP 1 257 333 A1. According to another aspect of the invention, the fiber bundle may consist of 80% to 95% crimped fibers and from 5% to 15% non-crimped fibers, relative to the total number of fibers in the bundle, for instance, from 86 to 94% crimped fibers and from 6 to 14% non-crimped fibers. In one embodiment, the proportion of crimped fibers is from 86 to 92%. The fibers have a sinusoidal texture with a wavelength in the range of from 6 to 9 mm, for instance, 7 to 8 mm; and an amplitude in the range of from 0.1 to 0.5 mm; for instance, 0.2 to 0.4 mm. Incorporation of 5 to 15% non-crimped fibers into a bundle of crimped semi-permeable hollow fiber membranes may enhance the performance of the hemodialyzer of the invention. For instance, with an unchanged packing density of the fibers within the dialyzer, the clearance of molecules like urea, vitamin B12, or cytochrome C from a fluid passing through the fiber lumen is increased. It is believed that this effect is due to improved flow of dialysis liquid in the second flow space of the dialyzer and around the individual fibers in the bundle. Another advantage of the incorporation of 5 to 15% non-crimped fibers into a bundle of crimped semi-permeable hollow fiber membranes is that packing densities can be achieved which are higher than those in bundles exclusively containing crimped fibers. As a consequence, a larger effective membrane area can be fitted into a given volume of the internal chamber of the hemodialyzer. Also, a given effective membrane area can be fitted into a smaller volume, which allows for further miniaturization of the hemodialyzer. Another alternative offered by the incorporation of 5 to 15% non-crimped fibers into a bundle of crimped semi-permeable hollow fiber membranes is that the crimp amplitude of the crimped fibers within the bundle can be increased at constant packing density and constant volume of the internal chamber, while the resilience of the bundle is kept at a value which does not require excessive force for the transfer of the bundle into the housing. This helps to avoid increased scrap rates in dialyzer production. When less than about 5% of non-crimped fibers are present in the bundle of semi-permeable hollow fiber membranes, no substantial difference in dialyzer performance is observed in comparison to a dialyzer comprising crimped fibers only. On the other hand, when more than about 15% of non-crimped fibers are present in the bundle, a decrease of dialyzer performance is noted. A potential explanation for this effect could be that, with increasing proportion of non-crimped fibers within the bundle, non-crimped fibers may contact and adhere to each other, thus reducing membrane surface area available for mass transfer through the hollow fiber walls.

The blood flow rates which can be used with devices comprising the membranes according to the invention are in the range of below 500 ml/min. Dialysate flow rates for use with the membranes according to the invention are in the range below 700 ml/min. Usually, blood flow rates of from 300 ml/min to 500 ml/min, dialysis flow rates of from 500 ml/min to 700 ml/min, preferably 500 ml/min and an UF rates of 0 ml/min is used. For example, a standard flow rate used is $Q_B$=300 ml/min, $Q_D$=500 ml/min and $Q_{UF}$=0ml/min.

Due to the combination of the membrane type, the physical properties of the single fibers and of the fiber bundle according to the invention, the hemodialyzers of the invention are especially beneficial for the treatment of chronic and acute renal failure by hemodialysis, thereby achieving and even exceeding a performance which can currently be achieved with prior art dialyzers. The new combined features allow the highly efficient removal of uremic molecules ranging from small to large molecular weight and also myoglobin, while efficiently retaining albumin and larger essential proteins.

This becomes especially apparent when considering the clearance performance of the hemodialyzers of the invention. The clearance Cl (ml/min) refers to the volume of a solution from which a solute is completely removed per time unit. In contrast to the sieving coefficient which is the best way to describe the structure and performance of a membrane as the essential component of a hemodialyzer, clearance is a measure of the overall dialyzer design and function and hence dialysis effectiveness. The clearance performance of a dialyzer can be determined according to DIN EN ISO8637:2014 For the present invention a modelling of mass transfer in membrane modules for blood purification was used for the comparison with prior art dialyzers. Clearance is used herein to describe the excellent performance which can be achieved by using the aforementioned highly efficient membranes in a hemodialyzer as described above.

With a hemodialyzer according to the invention, enhanced clearance rates may be accomplished in comparison with hemodialyzers according to prior art, while at the same time the required total membrane area ($A_{tot}$) (on blood side of membrane), and the required total volume (Vtot) (of the dialyzer housing) may be decreased.

For the comparison, prior art dialyzers were chosen based on maximal reduced Peclet$_{urea}$ number (at a blood flow ($Q_B$) of 500 ml/min). The membrane FXCorDiax80™ (Fresenius Medical Care), belonging to the high-flux type of membrane, and MCO-Ci 400 (Gambro Dialysatoren GmbH), belonging to the medium cut off type of membrane, was chosen for comparison with the dialyzers according to the invention as these two membranes have about the same Peclet$_{urea}$ number as the membrane according to the invention.

The two reference membranes were entered into the modelling of mass transfer in membrane modules and compared with two membranes according to the invention. Please see below results for entered parameters and results for Urea and Myoglobin clearance in Table III.

**TABLE III**: Comparison Prior Art Membranes and Inventive Membranes in Modelling of Mass Transfer

| Dialyzer/ Parameter | FXCorDiax 80 | MCO-Ci 400 | Inventive Membrane A | Inventive Membrane B |
|---|---|---|---|---|
| $\Delta P_B$ | <200 mmHg | <200 mmHg | <200 mmHg | <300 mmHg |
| $P_{B, out}$ | ≈100 mmHg | ≈100 mmHg | ≈100 mmHg | ≈100 mmHg |
| $P_{B, in}$ | <300 mmHg | <300 mmHg | <300 mmHg | <400 mmHg |
| PD | 0.602 | 0.56 | 0.598 | 0.598 |
| $H_{in}$ | 0.38 | 0.38 | 0.38 | 0.38 |
| $C_{p, in}$ | 70 g/L | 70 g/L | 70 g/L | 70 g/L |
| $d_H$ | 38.3 mm | 38 mm | 34.4 mm | 31.1 mm |

(continued)

| Dialyzer/ Parameter | FXCorDiax 80 | MCO-Ci 400 | Inventive Membrane A | Inventive Membrane B |
|---|---|---|---|---|
| $\delta_m$ | 39 $\mu$m | 35 $\mu$m | 25 $\mu$m | 25 $\mu$m |
| $d_B$ | 175 $\mu$m | 180 $\mu$m | 170 $\mu$m | 170 $\mu$m |
| $Q_{B, max}$ | 500 mL/min | 500 mL/min | 500 mL/min | 500 mL/min |
| $Q_{D, maX}$ | 700 mL/min | 700 mL/min | 700 mL/min | 700 mL/min |
| $Q_{UF, max}$ | 0 mL/min | 0 mL/min | 0 mL/min | 0 mL/min |
| $A_{tot}$ | 1.71 m$^2$ | 1.73 m$^2$ | 1.66 m$^2$ | 1.66 m$^2$ |
| L | 226 mm | 236 mm | 212 mm | 260 mm |
| L*N | 3105 km | 3059 km | 3107 km | 3107 km |
| $V_{tot}$ | 259 mL | 267.6 mL | 197.4 mL | 197.6 mL |
| $S_{myo}$ | 0.48 | 0.9 | 0.9 | 0.9 |
| $Pe_{r,max,urea}$ ($Q_B$=500ml/min) | 0.49 | 0.495 | 0.49 | 0.49 |
| $\alpha$ | 0.36 | 0.31 | 0.32 | 0.39 |
| $D_m/D_B$ | 0.11 (urea), 0.08 (myo) | 0.11 (urea), 0.36 (myo) | 0.11 (urea), 0.36 (myo) | 0.11 (urea), 0.36 (myo) |
| Urea Clearance | 287.85 mL/min | 288.72 mL/min | 319.17 mL/min | 329.61 mL/min |
| Myoglobin clearance | 54.29 mL/min | 95.44 mL/min | 112.23 mL/min | 129.85 mL/min |

According to above results, Inventive Membrane A, in comparison with FXCorDiax80, provides an increase by 10% of urea clearance and an increase by 104% of myoglobin clearance despite of a decrease by 3% of $A_{tot}$ and a decrease by 24% of $V_{tot}$. In comparison with MCO-Ci 400, Inventive Membrane A, provides an increase by 10% of urea clearance and an increase by 18% of myoglobin clearance despite of a decrease by 4% of $A_{tot}$ and a decrease by 26% of $V_{tot}$.

Further according to above results, Inventive Membrane B, in comparison with FXCorDiax80, provides an increase by 15% of urea clearance and an increase by 136% of myoglobin clearance despite of a decrease by 3% of $A_{tot}$ and a decrease by 24% of $V_{tot}$. In comparison with MCO-Ci 400, Inventive Membrane A, provides a, increase by 14% of urea clearance and an increase by 37% of myoglobin clearance despite of a decrease by 4% of $A_{tot}$ and a decrease by 4% of $V_{tot}$.

Thus, the hemodialyzers according to the invention may achieve increased clearance rates for urea and myoglobin in combination with a decrease in $A_{tot}$ and $V_{tot}$. Thus, an improved clearance is provided and at the same time the required total membrane area and the required housing volume is decreased.

It will be readily apparent to one skilled in the art that various substitutions and modifications may be made to the invention disclosed herein without departing from the scope and spirit of the invention.

The present invention will now be illustrated by way of non-limiting examples in order to further facilitate the understanding of the invention and provide information about the different test models used when investigating the properties of the membranes.

**Examples**

**Example 1**

Model validation

Figures 2a and 2b show the comparison between model-predicted and in vivo solute clearances obtained with FX CorDiax 80 (Fresenius Medical Care, Bad Homburg, Germany) (Figure 2a) and Revaclear MAX (Gambro Dialysatoren GmbH, Germany, a subsidiary of Baxter International Inc.) (Figure 2b), at increasing blood and dialysate flow rate, respectively. The clinical data are reported in Bhimani et al. in Effect of increasing dialysate flow rate on diffusive mass transfer of urea, phosphate and $\beta$2-microglobulin during clinical haemodialysis, Nephrol. Dial. Transplant. (2010) 1-6) and in Kirsch et al., in Performance of hemodialysis with novel medium cut-off dialyzers, Nephrol. Dial. Transplant. 0 (2016) 1-8, respectively. In both cases, good agreement between model-predicted and in vivo clearances was found at any flow rate for each solute, independent of solute molecular weight. In particular, the percentage error between model-predicted and in vivo clearance is lower than 7% for each solute at any simulated condition.

**Example 2**

Effect of membrane pressure modulus on solute clearance

Figure 3 shows the effect of the membrane pressure modulus, $\alpha$, on solute clearance at given module geometry and operating conditions. At lower values of $\alpha$, membrane resistance is high and solutes are mainly transported by diffusion. This causes solute clearance to be quasi-independent of the membrane pressure modulus. As the membrane pressure modulus increases for constant axial pressure drop in the blood and dialysate compartments, convective transport across the membrane is enhanced by the decreasing resistance to mass transport therein, which increases the forward ultrafiltration flow rate across the membrane and causes a slight increase of the solute clearances. This effect is slightly more evident for middle molecular weight solutes (i.e. myoglobin and $\beta$2-microglobulin).

**Example 3**

Effect of module geometry on solute clearance

Figures 4 and 5 show the effect of module geometry on dimensionless solute clearances at constant membrane pressure modulus and operating conditions. Figure 4 shows the effect of the fiber aspect ratio, $L/d_B$, on solute clearances. For each solute, dimensionless clearance increases with fiber aspect ratio. For low molecular weight solutes, dimensionless clearance increases up to a plateau (i.e. up to $C_{lj}^* = 1$). Figure 5 shows the effect of the module packing density, PD, on the dimensionless solute clearances. For each solute, dimensionless clearance increases as the module packing density increases. Both effects are mostly due to the increasing total module surface area.

**Claims**

1. A hemodialyzer for the purification of blood comprising a bundle of synthetic hollow fiber membranes, **characterized in that**

   (a) the hollow fiber membranes have an inner diameter (dB) within the range of 168-175 $\mu$m, a fiber wall thickness ($\delta_m$) within the range of 25-26 $\mu$m, and an effective length (L) within the hemodialyzer within the range of 207-287 mm;
   (b) the bundle of hollow fiber membranes has a packing density within the range of 57.5-60.0% and a total membrane surface area (Atot) within the range of from 1.64 to 1.73 $m^2$.

2. The hemodialyzer according to claim 1, wherein the hemodialyzer is further defined by having a membrane pressure modulus ($\alpha$) within the range of from 0.31 to 0.40.

3. The hemodialyzer according to claim 1 or claim 2, wherein the hemodialyzer is further defined by having a maximal reduced Peclet number for urea (Per,max, urea) in the range of from 0.45 to 0.49 at a blood flow ($Q_B$) of 500 ml/min and $D_b = 1.75e^{-9}$ $m^2/s$.

4. A hemodialyzer according to anyone of the preceding claims, wherein a housing of the dialyzer has an inner diameter (dH) within the range of 31-35 mm.

5. A hemodialyzer according to anyone of the preceding claims, wherein the membranes have a molecular retention onset (MWRO) of between 5 kDa and 20 kDa and a molecular weight cut-off (MWCO) of between 25 kDa and 320 kDa as determined by dextran sieving before blood contact to the membrane.

6. A hemodialyzer according to anyone of claims 1-4, wherein the membranes have a molecular retention onset (MWRO) of between 5 kDa and 10 kDa and a molecular weight cut-off (MWCO) of between 25 kDa and 65 kDa as determined by dextran sieving before blood contact to the membrane.

7. A hemodialyzer according to anyone of claims 1-4, wherein the membranes have a molecular retention onset (MWRO) of between 9 kDa and 14 kDa and a molecular weight cut-off (MWCO) of between 55 kDa and 130 kDa as determined by dextran sieving before blood contact to the membrane.

8. A hemodialyzer according to anyone of claims 1-4, wherein the membranes have a molecular retention onset (MWRO) of between 15 kDa and 20 kDa and a molecular weight cut-off (MWCO) of between 170 kDa and 320 kDa as determined by dextran sieving before blood contact to the membrane.

9. A hemodialyzer according to anyone of the preceding claims, wherein the housing of the hemodialyzer has an inner total volume (Vtot) within the range of 195 to 199 ml.

10. A hemodialyzer according to anyone of the preceding claims, wherein the fiber bundle consists of 80-95 % of crimped fibers and of 5% to 15% non-crimped fibers, relative to the total number of fibers in the bundle.

11. A hemodialyzer according to anyone of the preceding claims, wherein the hollow fiber membranes have an inner diameter (dB) within the range of 170-174 $\mu$m.

12. A hemodialyzer according to anyone of the preceding claims, wherein the hollow fiber membranes have a fiber wall thickness ($\delta_m$) of 25 $\mu$m.

13. A hemodialyzer according to anyone of the preceding claims, wherein the bundle of hollow fiber membranes has a packing density from 58.0 to 60.0%.

14. A hemodialyzer according to anyone of the preceding claims, wherein the hollow fiber membrane comprises at least one hydrophobic polymer and at least one hydrophilic polymer, wherein the at least one hydrophobic polymer is chosen from the group consisting of poly(aryl)ethersulphone (PAES), polypropylene (PP), polysulphone (PSU), polycarbonate (PC), polyarcrylonitrile (PAN), polyamide (PA), polytetrafluor¬ethylene (PTFE), polyethersulphone (PES), or combination thereof, and the at least one hydrophilic polymer is chosen from the group consisting of polyvinylpyrrolidone (PVP), polyethyleneglycol (PEG), polyvinylalcohol (PVA), and a copolymer of polypropyleneoxide and polyethyleneoxide (PPO-PEO) or combinations thereof.

15. A hemodialyzer according to anyone of claims 1-13, wherein the hollow fiber membrane comprises a copolymer of acrylonitrile and sodium methallyl sulfonate.

16. Use of a hemodialyzer according to anyone of the preceding claims for the purification of blood.

Figure 1

Figure 2a

Figure 2b

Figure 3

Figure 4

Figure 5

Figure 6 a

Figure 6 b

Figure 6 c

Figure 6 d

Figure 6 e

Figure 7

Europäisches
Patentamt

European
Patent Office

Office européen
des brevets

**EUROPEAN SEARCH REPORT**

Application Number

EP 17 16 6680

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 815 807 A1 (GAMBRO LUNDIA AB [SE]) 24 December 2014 (2014-12-24) * paragraphs [0001] - [0004], [0010], [0017], [0022] - [0029], [0035] * * claims 1-14 * * tables 1,2,3 * | 1-16 | INV. B01D61/28 B01D63/02 B01D69/08 A61M1/16 B01D61/24 |
| X | EP 3 102 312 A1 (GAMBRO LUNDIA AB [SE]) 14 December 2016 (2016-12-14) * claims 1-11 * * table 2 * * page 14 - page 16 * * page 23 - page 27 * * page 29 * * figures 1-7 * | 1-16 | ADD. B01D71/42 |
| X | Ashai Kasei Medical Co. Ltd: "Kf-201-m technical data sheet, EVAL EVOH", , 1 May 2015 (2015-05-01), XP055415576, Retrieved from the Internet: URL:http://www.asahi-kasei.co.jp/medical/en/pdf/dialysis/kf-201-m_catalog.pdf [retrieved on 2017-10-13] * whole document, in particular: * * tables 1,2 * | 1-14,16 | |
| X | Ashai Kasei Medical Group Ltd: "KF-201-C Hollow fibre dialyzer EVAL EVOH", , 1 May 2015 (2015-05-01), XP055415581, Retrieved from the Internet: URL:http://www.asahi-kasei.co.jp/medical/en/pdf/dialysis/kf-201-c_catalog.pdf [retrieved on 2017-10-13] * whole document in particular: * * tables 1,2 * | 1-14,16 | |

TECHNICAL FIELDS
SEARCHED      (IPC)

B01D
A61M

The present search report has been drawn up for all claims

| Place of search | Date of completion of the search | Examiner |
|---|---|---|
| Munich | 17 October 2017 | Williams, Jennifer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&: member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 6680

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2017

| Patent document cited in search report | | Publication date | Patent family member(s) | | Publication date |
|---|---|---|---|---|---|
| EP 2815807 | A1 | 24-12-2014 | CN | 104394967 A | 04-03-2015 |
| | | | EP | 2815807 A1 | 24-12-2014 |
| | | | EP | 3010629 A1 | 27-04-2016 |
| | | | ES | 2623282 T3 | 10-07-2017 |
| | | | JP | 2016528948 A | 23-09-2016 |
| | | | PL | 3010629 T3 | 31-08-2017 |
| | | | US | 2016129172 A1 | 12-05-2016 |
| | | | WO | 2014202710 A1 | 24-12-2014 |
| EP 3102312 | A1 | 14-12-2016 | AU | 2015214950 A1 | 04-08-2016 |
| | | | CA | 2938222 A1 | 13-08-2015 |
| | | | CN | 105722582 A | 29-06-2016 |
| | | | EP | 3102312 A1 | 14-12-2016 |
| | | | HK | 1224246 A1 | 18-08-2017 |
| | | | JP | 2017507706 A | 23-03-2017 |
| | | | KR | 20160119190 A | 12-10-2016 |
| | | | US | 2017165615 A1 | 15-06-2017 |
| | | | WO | 2015118046 A1 | 13-08-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0844015 A2
- EP 0305687 A1
- WO 0160477 A2
- US 5891338 A
- EP 2113298 A
- WO 2004056460 A1
- EP 2161072 A1

- US 20120305487 A1
- US 7875183 B2
- WO 2015118045 A
- WO 2015118046 A
- WO 2013190022 A1
- EP 1257333 A1

**Non-patent literature cited in the description**

- **WARD.** Ward RA. Protein-leaking membranes for hemodialysis: a new class of membranes in search of an application?. *J Am Soc Nephrol.,* 2005, vol. 16 (8), 2421-2430
- **GRANATH et al.** Molecular weight distribution analysis by gel chromatography on sephadex. *J Chromatogr A,* 1967, vol. 28 (C), 69-81
- *J. Am. Soc. Nephrol.,* 2007, 18
- Artieries and arterial blood flow:biological and physiological aspects. General features of blood circulation in narrow vessels. Springer-Verlag, 1983
- **BIRD et al.** Transport phenomena. J Wiley & Sons, inc, 2002
- **BRINKMAN, A.** calculation of the viscos force exerted by a flowing fluid on a dense swarm of particles. *Appl. Sci. Res. A,* 1947, vol. 1, 27-34
- **ELOOT et al.** Optimisation of solute transport in dialysers using a three-dimensional finite volume model. *Comp. Meth, Biomed. Eng.,* 2006, vol. 9, 363-370
- **ZYDNEY.** Bulk mass transport limitations during high flux hemodialysis. *Artif. Org.,* 1993, vol. 17, 919-924

- Arteries and arterial blood flow: biological and physiological aspects. **QUEMADA.** General features of blood circulation in narrow vessels. Springer-Verlag, 1983
- **R.B. BIRD et al.** Transport phenomena. J. Wiley & Sons, Inc, 2002
- **R.L. FOURNIER.** Basic Transport Phenomena in Biomedical Engineering. CRC Press, 2012
- **A. APELBLAT et al.** A mathematical analysis of capillary tissue fluid exchange. *Biorheology,* 1974, vol. 11, 1-49
- **WARD ; OUSEPH.** Modification of membrane characteristics allows a reduction in dialyzer membrane area without loss of performance. *J. Am. Soc. Nephrol.,* 2007, vol. 18
- **BHIMANI et al.** Effect of increasing dialysate flow rate on diffusive mass transfer of urea, phosphate and $\beta$2-microglobulin during clinical haemodialysis. *Nephrol. Dial. Transplant.,* 2010, 1-6
- **KIRSCH et al.** Performance of hemodialysis with novel medium cut-off dialyzers. *Nephrol. Dial. Transplant.,* 2016, vol. 0, 1-8